(19) Europäisches Patentamt
European Patent Office
Office européen des brevets

(11) **EP 2 710 113 B1**

(12) **FASCICULE DE BREVET EUROPEEN**

(45) Date de publication et mention de la délivrance du brevet:
**28.12.2016 Bulletin 2016/52**

(21) Numéro de dépôt: **12721539.0**

(22) Date de dépôt: **18.05.2012**

(51) Int Cl.:
*C12N 1/12* (2006.01)      *C12P 5/00* (2006.01)
*C12P 7/64* (2006.01)      *A23L 29/00* (2016.01)
*A23L 33/10* (2016.01)     *A61K 31/01* (2006.01)
*A61K 31/202* (2006.01)    *C12R 1/89* (2006.01)

(86) Numéro de dépôt international:
**PCT/EP2012/059231**

(87) Numéro de publication internationale:
**WO 2012/159980 (29.11.2012 Gazette 2012/48)**

(54) **NOUVELLE SOUCHE DE MICROALGUE PRODUCTRICE DE SQUALENE**

NEUER SQUALEN-PRODUZIERENDER STAMM AUS MIKROALGEN

NOVEL STRAIN OF MICROALGA THAT PRODUCES SQUALENE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.05.2011 CN 201110147066**

(43) Date de publication de la demande:
**26.03.2014 Bulletin 2014/13**

(73) Titulaire: **Roquette Frères**
**62136 Lestrem (FR)**

(72) Inventeurs:
• **PORA, Bernard**
  **Dongfeng Sunshine City**
  **Wuhan (Economic Develpt Area) 430056 (CN)**
• **ZHOU, Jie**
  **Wuhan 430056 (CN)**
• **DEFRETIN, Sophie**
  **F-62400 Bethune (FR)**
• **VANDEWALLE, Xavier**
  **F-59114 Steenvoorde (FR)**

(74) Mandataire: **Gallois, Valérie et al**
**Cabinet BECKER & ASSOCIES**
**25, rue Louis Le Grand**
**75002 Paris (FR)**

(56) Documents cités:
**WO-A1-2012/077799**

• **Atsushi Nakazawa et al: "Optimisation of culture conditions of thraustochytrid, Aurantiochytrium sp. strain 18W-13a for squalene production", University of Tsukuba THE 1ST ASIA OCEANIA ALGAE INNOVATION SUMMIT, 13 décembre 2010 (2010-12-13), 14 décembre 2010 (2010-12-14), pages front-page 70, XP002681839, Tsukuba, Japan Extrait de l'Internet: URL:http://www.sakura.cc.tsukuba.ac.jp/~eeeforum/AOAIS/YT_final%204.pdf [extrait le 2012-08-15]**
• **TSUI C K M ET AL: "Labyrinthulomycetes phylogeny and its implications for the evolutionary loss of chloroplasts and gain of ectoplasmic gliding", MOLECULAR PHYLOGENETICS AND EVOLUTION, ORLANDO, FL, US, vol. 50, no. 1, 1 janvier 2009 (2009-01-01), pages 129-140, XP025804364, ISSN: 1055-7903, DOI: 10.1016/J.YMPEV.2008.09.027 [extrait le 2008-10-17] -& DATABASE NUCLEOTIDE [Online] 30 septembre 2006 (2006-09-30), "Schizochytrium sp. ATCC 20888 18S ribosomal RNA, partial sequence.", XP002681840, extrait de NCBI Database accession no. DQ367050**
• **LI QIAN ET AL: "Screening and Characterization of Squalene-Producing Thraustochytrids from Hong Kong Mangroves", JOURNAL OF AGRICULTURAL AND FOOD CHEMISTRY, vol. 57, no. 10, mai 2009 (2009-05), pages 4267-4272, XP002681841, ISSN: 0021-8561**

EP 2 710 113 B1

- **JIANG YUE ET AL: "Fatty acid composition and squalene content of the marine microalga Schizochytrium mangrovei.", JOURNAL OF AGRICULTURAL AND FOOD CHEMISTRY, vol. 52, no. 5, 10 mars 2004 (2004-03-10), pages 1196-1200, XP002681842, ISSN: 0021-8561**
- **KUNIMITSU KAYA ET AL: "Thraustochytrid Aurantiochytrium sp 18W-13a Accummulates High Amounts of Squalene", BIOSCIENCE BIOTECHNOLOGY BIOCHEMISTRY, vol. 75, no. 11, 1 novembre 2011 (2011-11-01), pages 2246-2248, XP002678626, JAPAN SOCIETY FOR BIOSCIENCE, BIOTECHNOLOGY, AND AGROCHEMISTRY, TOKYO, JAPAN ISSN: 0916-8451, DOI: 10.1271/BBB.110430 [extrait le 2011-11-07]**
- **ATSUSHI NAKAZAWA ET AL: "Optimization of culture conditions of the thraustochytrid Aurantiochytrium sp. strain 18W-13a for squalene production", BIORESOURCE TECHNOLOGY, vol. 109, 1 avril 2012 (2012-04-01), pages 287-291, XP002678627, ELSEVIER BV, GB ISSN: 0960-8524, DOI: 10.1016/J.BIORTECH.2011.09.127 [extrait le 2011-10-07]**

Remarques:

Le dossier contient des informations techniques présentées postérieurement au dépôt de la demande et ne figurant pas dans le présent fascicule.

**Description**

La présente invention se rapporte à une nouvelle souche de microalgues particulièrement adaptée à la production de squalène et à ses utilisations.

Le squalène est un triterpène, isoprénoïde à trente atomes de carbone et cinquante atomes d'hydrogène, de formule : 2,6,10,15,19,23-Hexaméthyl-2,6,10,14,18,22-tétracosahexène.

C'est un lipide naturellement produit par tous les organismes supérieurs y compris chez l'Homme (retrouvé dans le sébum). Le squalène est en effet un intermédiaire essentiel dans la biosynthèse du cholestérol, des hormones stéroïdes et de la vitamine D (une enzyme des voies métaboliques du cholestérol, la squalène monooxygénase, en oxydant l'une des extrémités de la molécule de squalène, va induire sa cyclisation et conduire au lanostérol, lequel sera transformé en cholestérol et en autres stéroïdes).

En industrie, le squalène est surtout utilisé dans les domaines alimentaire, cosmétique et pharmaceutique.

Comme complément alimentaire, le squalène est habituellement formulé en capsules ou en huiles.

Dans le domaine cosmétique, cette molécule peut être utilisée comme antioxydant, antistatique et émollient dans les crèmes hydratantes, pénétrant rapidement la peau sans laisser de traces ou de sensations grasses, et se mélangeant bien avec d'autres huiles et vitamines.

Dans ce domaine, notons qu'étant donné la très grande instabilité du squalène (6 insaturations), c'est la forme saturée squalane (obtenue par hydrogénation), meilleur antioxydant que le squalène, que l'on trouve sur le marché, et à niveau de pureté en général très élevé (99%).

Les études toxicologiques ont montré qu'aux concentrations utilisées dans les cosmétiques, le squalène et le squalane ne présentent pas de toxicité, et ne sont pas irritants ou sensibilisants pour la peau humaine.

Dans le domaine pharmaceutique, le squalène est utilisé comme adjuvants aux vaccins.

Ces adjuvants sont des substances qui stimulent le système immunitaire et augmentent la réponse au vaccin.

Sous la forme d'une émulsion ajoutée à la substance vaccinante pour rendre le vaccin plus immunogène, du squalène est utilisé depuis 1997 dans un vaccin antigrippal

*(FLUAD,* de la société CHIRON, contre la grippe saisonnière) à raison d'environ 10 mg de squalène par dose.

Comme tous les vaccins contenant du squalène, ces émulsions ont un aspect blanc laiteux.

Le squalène est également utilisé comme adjuvant vaccinal, notamment des vaccins expérimentaux, substances antipaludéennes ou vaccin antigrippe ciblant les virus émergents H5N1 puis en 2009 H1N1, en tant que :

- constituant breveté du système adjuvant AS03 utilisé par la société GLAXOSMITHKLINE dans le vaccin contre la pandémie grippale 2009 Pandemrix et Arepanrix,
- constituant breveté du système adjuvant MF59 utilisé par la société NOVARTIS.

Le squalène a été également ajouté aux vaccins antigrippaux pour stimuler la réponse immunitaire du corps humain à travers la production de cellules CD4 à mémoire.

C'est le premier adjuvant huile-dans-l'eau pour vaccin contre la grippe à avoir été commercialisé en association avec les antigènes du virus de la grippe saisonnière.

Le niveau de pureté du squalène est essentiel dans ce domaine d'applications.

En effet, s'il est pris par voie orale, le squalène est considéré comme totalement sûr ; mais c'est la voie injectable qui fait l'objet de controverses.

En effet, dans le domaine médical, le risque de préjudice pour un receveur humain peut être accru dans les situations où le squalène est contaminé par des impuretés, car par définition, cet adjuvant peut induire une forte réponse immunitaire également contre ses propres impuretés.

Il est donc indispensable de disposer de squalène de haute qualité, exempt d'impuretés (traces de métaux, notamment de mercure, et d'autres toxines).

Un certain nombre de voies de production du squalène sont proposées dans la littérature.

C'est un composé que l'on trouve souvent stocké dans le foie des poissons cartilagineux tels que les requins des profondeurs (d'où son nom).

Il est donc une des causes de leur surpêche, le requin étant déjà chassé pour ses ailerons. Les foies de requin sont ainsi désormais vendus pour produire des gélules qualifiées de "bonnes pour la santé".

Cependant, si le squalène commercialisé est ainsi principalement extrait de foies de requins, il n'est pas exempt de problème sanitaire.

En effet, les requins peuvent être infectés par des pathogènes pouvant produire des substances nuisibles pour l'homme. En outre, le foie des requins, organe d'élimination et de purification de l'organisme, peut contenir des toxines telles que la carchatoxine qui est néfaste pour l'homme.

Ces préoccupations environnementales (forte régression des requins) et sanitaires (le foie des poissons stocke aussi des toxines préoccupantes pour la santé) ont motivé son extraction à partir de végétaux.

Il est ainsi possible de l'isoler de l'huile d'olive, l'huile de palme, et dans d'autres huiles céréalières ou provenant de l'amarante, des semences, du son de riz, de germes de blé.

Cependant, l'inconvénient majeur est ici que le squalène est extrait en très faibles quantités, de l'ordre de 0,1 à 0,7 % en poids.

En première alternative à ces procédés d'extraction à partir de foies de requins ou de végétaux, souvent rendus coûteux par la mise en oeuvre de procédés d'enrichissement et de purification importants, ont été proposés de premiers procédés de production de squalène à partir de microorganismes : levures naturelles ou levures recombinantes, notamment de type *Saccharomyces.*

C'est ainsi que Saccharomyces cerevisiae est connue pour sa capacité à produire du squalène, cependant en très faible quantité : de l'ordre de 0,041 mg/g de biomasse (BHATTACHARJEE, P. et al, 2001, dans World J. Microb. Biotechnol., 17, pp 811-816).

L'optimisation de ces capacités de production a donc été travaillée, par le biais de la recombinaison génétique.

Les levures recombinantes qui produisent du squalène ont ainsi l'avantage :

- de bénéficier du même statut GRAS (*Generally Regarded As Safe*) que la cellule hôte,
- d'être exemptes de pathogènes, de prions ou de toxines, tout comme la cellule hôte, et
- d'avoir déjà été utilisées dans le domaine des vaccins (telles que ces levures qui expriment des vecteurs contenant des antigènes de l'hépatite B).

Cependant, comme le présente la demande de brevet WO 2010/023551 pour le domaine médical (production de squalène d'une pureté supérieure à 97 % comme adjuvant de vaccins), cette première alternative n'est industrialisable que si l'on peut disposer de levures recombinantes hyperproductrices de squalène (à plus de 15 % en poids de cellules sèches).

Or, l'obtention de ces cellules recombinantes nécessite la mise en oeuvre de nombreuses étapes lourdes, longues et complexes d'ingénierie métabolique, par la mise en oeuvre d'outils de biologie moléculaire, conduisant à la stimulation des voies de biosynthèse du squalène et à l'inhibition des voies du catabolisme du squalène.

En effet, comme le rappelle d'ailleurs ladite demande de brevet WO 2010/023551, les gènes impliqués dans la biosynthèse du squalène sont multiples : incluant la mévalonate kinase, la phosphomevalonate kinase, la pyrophosphomevalonate décarboxylase, l'isopentenyl pyrophosphate isomérase, la HMGR (3-hydroxy-3-methylglutaryl-CoA réductase), et la squalène synthétase.

Pour les voies du catabolisme, les gènes codant pour de nombreuses enzymes impliquées dans la conversion du squalène en ergostérol incluant la squalène époxidase (ERGI), la lanostérol synthétase, la C14-diméthylase, la dl4-réductase, la C4-méthyloxidase, la C4-décarboxylase (ERG26), la 3-cétoréductase, la C24-méthyltransférase, la C8-isomérase, la C5-désaturase, la d22-désaturase et la d24-réductase.

Par ailleurs, d'autres enzymes du catabolisme doivent être également considérées : la LEU2 ([beta]-isopropylmalate déshydrogénase), l'oxydosqualène cyclase, la zymostérol-24-méthyltransférase et l'ergosta-5,7,24(28)-trienol-22- déshydrogénase.

En deuxième alternative aux procédés d'extraction à partir de foies de requins ou de végétaux, ont été proposés des procédés prometteurs de production de squalène à partir de microalgues de la famille des Thraustochytriales (comprenant les genres *Thraustochytrium, Aurantiochytrium* et *Schizochytrium),* plus particulièrement *Schizochytrium mangrovei* ou *Schizochytrium limacinum.*

Ces microalgues produisent du squalène en conditions hétérotrophiques (absence de lumière ; apport de glucose comme source carbonée), et peuvent donc être manipulées aisément par l'homme du métier du domaine de la fermentation de microorganismes.

Ces procédés offrent donc, par le biais de conditions de fermentation contrôlées, des qualités de squalène dont la purification est aisément réalisable pour répondre aux besoins alimentaires, cosmétiques et médicales.

Chez ces microalgues de la famille des Thraustochytriales, le squalène est cependant le coproduit d'autres composés lipidiques d'intérêt, tel l'acide docosahexaénoïque (ou DHA), acide gras polyinsaturé de la famille des ω3.

Il apparaît ainsi que le squalène est surtout décrit comme l'un des composants de la fraction insaponifiable des huiles commerciales de DHA (à côté des caroténoïdes et des stérols).

A titre de comparaison, la souche de *Schizochytrium mangrovei* FB1 produit du DHA à raison de 6,2 % en poids sec de cellules, pour 0,017 % de squalène.

De ce fait, ces microorganismes qui produisent naturellement du squalène le font en faibles quantités :

- de l'ordre de 0,1 mg/g de biomasse, pour Thraustochytrid ACEM 6063 (cf. LEWIS et al dans Mar. Biotechnol., 2001, 439-447),
- de l'ordre de 0,162 mg/g de biomasse, pour *Schizochytrium mangrovei* FB1 (cf. JIANG et al, dans J. Agric. Food Chem., 2004, 52, pp 1196-1200),

- de l'ordre de 0,18 mg/g de biomasse, pour un isolat de mangroves de Hong Kong *Aurantiochytrium BR-MP4-A1* (cf. LI et al, dans J. Agric. Food Chem., 2009, 57; pp 4267-4272).

Pour augmenter ces productions, il est alors apparu indispensable d'optimiser les conditions de fermentation.

Dans l'article de QIAN Li et al, dans J. Agric. Food Chem., 2009, 57, 4267-4272, il est précisé que le squalène est un intermédiaire clef de la biosynthèse des stérols, et que la première étape de la conversion du squalène en stérols est catalysée par une squalène époxidase, dépendante de l'oxygène.

Des conditions riches en oxygène dissous sont donc à proscrire si l'on souhaite au contraire accumuler le squalène intracellulaire.

C'est ainsi que le fait de cultiver Thraustochytride ACEM 6063 à niveau faible en oxygène dissous (0 à 5% de saturation) permet d'accumuler plus de 1 mg/g de squalène, alors que la croissance à plus haut niveau d'oxygène dissous (40 à 60 %) ne permet d'atteindre que 0,01 mg/g de squalène.

De la même manière, une culture à température de 15°C place la production de squalène par Thraustochytride ACEM 6063 à 1,2 mg/g, alors qu'elle n'est que de 0,7 mg/g à 20°C (cf. LEWIS et al, dans Mar. Biotechnol., 2001, 3, 439-447*).*

Dans l'article de G. CHEN et al., dans New Biotechnology, 2010, 27-4, pp 382-389, il est rappelé que *Schizochytrium* produit principalement du DHA, au travers de la voie des polykétides synthétases (acronyme : PKS), tandis que le squalène est plutôt synthétisé au travers de la voie de biosynthèse du cholestérol, ce qui signifie que les besoins nutritionnels des thrautochytrides pour ces deux composés sont distincts.

L'objet de leur travail a donc été de rechercher de manière systématique l'effet de différentes sources d'azote dans la production de squalène.

G. CHEN et al ont ainsi trouvé que *Schizochytrium* pouvait croître rapidement et accumuler des quantité "élevées" en squalène dans un milieu de culture contenant un mélange de sources azotées constitué de glutamate monosodique, d'extrait de levures et de tryptone.

Malgré cela, cette production "élevée" en squalène est toute relative.

En effet, si les auteurs réussissent à augmenter significativement le contenu en squalène et le rendement de 26,3 % et 10,1 %, respectivement, par rapport aux valeurs du milieu de base, ces conditions optimisées conduisent en fait à un contenu en squalène de 0,72 mg/g et un titre de 5,90 mg/l.

Avec ce même objectif d'optimiser la production de squalène, K. W. FAN et al, dans World J. Microbiol. Biotechnol., 2010, 26-3, pp 1303-1309, ont utilisé un inhibiteur de la squalène monooxygénase (enzyme clef de la biosynthèse des stérols) : la terbinafine hydroxychloride.

Il est connu que le contenu et le rendement en squalène sont liés à l'âge de la culture des microorganismes.

Plus la culture cellulaire vieillit, moins elle accumule du squalène ; en fait plus elle en consomme dans la voie de biosynthèse des stérols.

La terbinafine agit donc en empêchant cette consommation du squalène vers la voie des stérols et permet alors de stimuler l'accumulation intracellulaire de squalène jusqu'à 36 à 40 % par rapport au contrôle.

Mais la production la plus élevée obtenue en squalène avec la souche *d'Aurantiochytrium mangrovei* FB3 utilisée dans cette étude, même si bien supérieure à celle décrite pour S. *cerevisiae* (0,041 mg/g de biomasse), voire même celle décrite pour *Torulaspora debrueckii* (0,24 mg/g de biomasse), n'est que de 0,53 mg/g de biomasse.

Ainsi, malgré tous les efforts déployés, ces valeurs sont bien inférieures à celles de référence pour l'huile d'olive (de l'ordre de 4,24 mg/g) et sont éloignées des valeurs requises à l'échelle industrielle.

Nakazawa et al (« Optimisation of culture conditions of thraustochytrid, Aurantiochytrium sp. Strain 18W-13a for squalene production », University of Tsukuba, The 1 st Asia Oceania algae innovation Summit, 13 décembre 2010) décrit une souche de *Aurantiochytrium* appelée 18W-13A appartenant à la famille des *Thraustochytriaceae.*

Tsui et al (2009, Molecular Phylogenetics and Evolution, 50, 129-140) décrit une souche de *Schizochytrium* ATCC 2088.

Qian et al (2009, Journal of Agricultural and Food Chemistry, 57, 4267-4272) décrit l'utilisation de squalène dans la préparation de compositions pharmaceutiques et alimentaires.

**Soucieuse de mettre au point un procédé de production bien plus efficace et bien moins couteux que ceux décrits dans l'état de la technique,** la société Demanderesse a, au cours de ses recherches, identifié une nouvelle souche de microalgues présentant une capacité exceptionnelle de production de squalène puisqu'elle permet une production de plus de 10 mg de squalène par g de biomasse, par exemple 18 mg de squalène par g de biomasse, soit 30 fois plus que la production la plus élevée obtenue en squalène avec la souche *d'Aurantiochytrium mangrovei* FB3. Par ailleurs, outre la capacité remarquable de production de squalène, cette souche permet également d'obtenir d'autres composés lipidiques d'intérêt tel que l'acide docosahexaénoïque (ou DHA) à plus de 15 % en poids sec de biomasse, par exemple 17,6 % en poids sec de biomasse.

Cette souche de *Schizochytrium sp.* a été déposée en France le 14 avril 2011 auprès de la Collection Nationale de Cultures de Microorganismes de l'Institut Pasteur (CNCM) sous le numéro I-4469 mais également en Chine auprès du CHINA CENTER FOR TYPE CULTURE COLLECTION (CCTCC) de l'université de Wuhan, Wuhan 430072, P.R. China sous le numéro M 209118. Elle a été caractérisée par séquençage partiel du gène codant pour l'ARN 18S (SEQ ID No 1):

```
  1 GAGGGTTTTA CATTGCTCTC aTTCCaATAG CAaGACGCGA AGCGCCCCGC ATTGATATTT
 61 CTCGTCACTA CCTCGTGGAG TCCACATTGG GTAATTTACG CGCCTGCTGC CTTCCTTGGA
121 TGTGGTAGCC GTCTCTCAGG CTCCCTCTCC GGAGTCGAGC CCTAACTCCC CGTCACCCGT
181 TATAGTCACC GTAGGCCAAT ACCCTACCGT CGACAACTGA TGGGGCAGAA ACTCAAACGA
241 TTCATCGCTC CGAAAAGCGA TCTGCTCAAT TATCATGACT CACCAAGAGA GTTGGCTTAG
301 ACCTAATAAG TGCGGCCCTC CCCGAAAGTC GGGCCCGTAC AGCACGTATT AATTCCAGAA
361 TTACTGCAGG TATCCGTATA AAGGAACTAC CGAAGGGATT ATAACTGATA TAATGAGCCG
421 TTCGCAGTTT CACAGTATAA TTCGCTTATA CTTACACATG CATGGCTTAG TCTTTGAGA
```

ce qui a permis de l'identifier comme étant une souche du type *Schizochytrium sp.*

Par conséquent, la présente invention est relative à la souche de *Schizochytrium* sp. déposée le 14 avril 2011 auprès de la CNCM sous le numéro I-4469. Cette souche pourra être désignée « CNCM I-4469 » ultérieurement dans la présente demande.

Cette souche présente la propriété intéressante de produire du squalène en grande quantité. En effet, elle permet l'obtention de squalène à l'échelle du gramme pour 100 grammes de biomasse sèche. En particulier, la quantification de squalène produit peut être réalisée selon la méthode détaillée dans la partie expérimentale.

C'est pourquoi la présente description décrit également un variant de cette souche ou une souche dérivée de celle-ci, ledit variant ou ladite souche dérivée conservant la propriété de produire des teneurs en squalène supérieures ou égales à 1 g pour 100 g de biomasse sèche. En particulier, elle est relative à une souche de *Schizochytrium sp.* capable de produire des teneurs en squalène supérieures ou égales à 1 g pour 100 g de biomasse sèche, et obtenue à partir de la souche CNCM I-4469 par mutagenèse ou par transformation génique. La mutagenèse peut être dirigée et/ou aléatoire.

La présente description décrit aussi une méthode de préparation d'une telle souche comprenant la mutagenèse ou la transformation génique de la souche CNCM I-4469 et facultativement une étape de criblage permettant de sélectionner les souches produisant au moins 1 g de squalène pour 100 g de biomasse sèche.

L'invention concerne une méthode de culture de la souche CNCM I-4469 ou d'un variant de celle-ci conservant la capacité de production de squalène, comprenant une étape de culture de cette souche dans un milieu approprié et une étape de récupération de la biomasse. La culture est réalisée en conditions hétérotrophiques. Généralement, l'étape de culture comprend une étape de préculture, pour revivifier la souche, puis une étape de culture ou de fermentation proprement dite. Cette dernière étape correspond à l'étape de production des composés lipidiques d'intérêt.

Les conditions de culture de ces microalgues sont bien connues dans le domaine. Par exemple dans l'article de G. CHEN cité *supra,* on trouve un procédé comprenant les étapes successives suivantes :

- partir de la souche maintenue sur milieu nutritif gélosée comprenant du glucose, du glutamate monosodique, de l'extrait de levures et des oligoéléments divers,
- réaliser une préculture en Erlenmeyers sur agitateur orbital, à un pH de 6, à une température de 25°C afin d'obtenir une biomasse revivifiée,
- ensemencer une autre série d'Erlenmeyers de production, avec le même milieu de culture que celui utilisé en préculture, avec de l'ordre de 0,5 % (v/v) de la biomasse obtenue à l'étape précédente, et maintenir la température à 25°C.

La préculture peut durer de préférence de 24 à 74 heures, de préférence environ 48 heures. La culture quant à elle peut durer de préférence de 60 à 150 heures.

La source carbonée nécessaire à la croissance de la microalgue est préférentiellement du glucose.

Quant à la nature de la source d'azote, la société Demanderesse a trouvé qu'il est possible de la choisir dans le groupe constitué des extraits de levure, de l'urée, du glutamate de sodium, du sulfate d'ammonium pris seuls ou en combinaison. De la même façon, il est possible de remplacer l'urée par du glutamate de sodium en tout ou partie, ou utiliser un mélange de glutamate de sodium et de sulfate d'ammonium.

Il est possible de préférer aux extraits de levure, classiquement mis en oeuvre dans les procédés de l'état de la technique, de l'urée complétée par un cocktail de vitamines, telle le cocktail BME commercialisé par la société SIGMA, utilisé à raison de 5 ml/l.

De préférence, les milieux de préculture comprennent des vitamines B1, B6 et B12.

Quant au pH du milieu de culture, comme il sera exemplifié ci-après, il sera maintenu entre 5,5 et 6,5, préférentiellement fixé à une valeur de 6. La régulation du pH peut se faire par tout moyen connu de l'homme du métier, par exemple par ajout d'acide sulfurique 2 N, puis avec de la soude 8 N.

Enfin, le taux d'oxygène dissous peut être régulé à une valeur comprise entre 20 et 0 %, de préférence maintenu à 5 % pendant une durée initiale de 24 et 48 heures, de préférence 36 heures, avant d'être laissée à 0 %. Quant au transfert d'oxygène, il sera régulé par tout moyen connu par ailleurs de l'homme du métier, de manière à ne pas dépasser 45 mmoles/l/heure.

L'invention concerne tout particulièrement l'utilisation de la souche CNCM 1-4469 ou d'un variant de celle-ci conservant la capacité de production de squalène, pour la production de composés lipidiques d'intérêt. Les composés lipidiques d'intérêt comprennent le squalène et notamment l'acide docosahexaénoïque (ou DHA). De préférence, le composé d'intérêt est le squalène. Il est à noter que le squalène peut être produit avec des teneurs supérieures ou égales à 1 g pour 100 g de biomasse sèche.

L'invention concerne une méthode de production de composés lipidiques d'intérêt comprenant la culture de la souche CNCM I-4469 ou d'un variant de celle-ci conservant la capacité de production de squalène et la récupération de la biomasse riche en composés lipidiques d'intérêt et, facultativement, la récolte et/ou la purification des composés lipidiques d'intérêt. Les composés lipidiques d'intérêt comprennent le squalène et notamment l'acide docosahexaénoïque (ou DHA). De préférence, le composé d'intérêt est le squalène. En particulier, le squalène peut être produit avec des teneurs supérieures ou égales à 1 g pour 100 g de biomasse sèche.

Après l'étape de fermentation, la biomasse peut être récupérée du milieu de fermentation par toute méthode connue en soi de l'homme du métier, par exemple la biomasse peut être extraite du fermenteur et simplement concentrée par microfiltration ou centrifugation, ou lavée par succession de concentrations-dilutions avec une solution aqueuse.

L'invention concerne ainsi la biomasse comprenant la souche CNCM I-4469 ou d'un variant de celle-ci conservant la capacité de production de squalène. Tout particulièrement, après l'étape de fermentation ou culture, cette biomasse est riche en composés lipidiques d'intérêt tels que le squalène et l'acide docosahexaénoïque, de préférence le squalène. Elle est susceptible d'être obtenue par la méthode décrite dans le présent document. En effet, après fermentation, la biomasse peut contenir 17,6 % en poids d'acide docosahexaénoïque et 1,8 % en poids de squalène. Par « riche en squalène » est entendu ici une teneur supérieure ou égale à 1 g pour 100 g de biomasse sèche.

Outre la biomasse, la présente déscription décrit également un extrait ou lysat cellulaire préparé à partir de cette biomasse comprenant la souche CNCM I-4469 ou d'un variant de celle-ci conservant la capacité de production de squalène. En particulier, cet extrait ou lysat est préparé à partir de la biomasse récupérée après fermentation. Cet extrait ou lysat riche en composés lipidiques d'intérêt tels que le squalène et l'acide docosahexaénoïque, de préférence le squalène. La rupture des cellules pour l'extraction du contenu lipidique peut être effectuée par différentes voies parmi lesquelles les voies mécanique, chimique, enzymatique.

Par la suite, l'huile peut être extraite du lysat cellulaire à l'hexane/éthanol en plusieurs extractions successives. La fraction hexanique est ensuite séparée puis l'hexane est évaporé pour isoler l'huile brute.

Ainsi, la méthode de production de composés lipidiques d'intérêt, de préférence le squalène, comprend la récolte de la biomasse, la préparation d'un extrait ou lysat cellulaire et extraction d'une huile brute comprenant les composés lipidiques d'intérêt, de préférence le squalène.

La présente description décrit également une huile brute ou raffinée comprenant les composés lipidiques d'intérêt, de préférence le squalène, préparée à partir de cette biomasse comprenant la souche CNCM I-4469 ou d'un variant de celle-ci conservant la capacité de production de squalène.

La présente description décrit finalement l'utilisation des composés lipidiques d'intérêt, tel le squalène ou l'acide docosahexaénoïque (ou DHA), en particulier le squalène, produits par l'un quelconque des procédés de la présente invention dans la préparation de compositions destinées aux domaines médicaux, cosmétiques et alimentaires. Ainsi, elle concerne une méthode de préparation de compositions destinées aux domaines médicaux, cosmétiques et alimentaires comprenant la production des composés lipidiques d'intérêt, tel le squalène ou l'acide docosahexaénoïque (ou DHA), en particulier le squalène, par l'un quelconque des procédés de la présente invention puis la préparation de compositions destinées aux domaines médicaux, cosmétiques et alimentaires.

La présente description est relative en particulier à un produit ou une composition comprenant la souche CNCM I-4469 ou d'un variant de celle-ci conservant la capacité de production de squalène, une biomasse obtenue après culture ou fermentation de celle-ci, un extrait ou lysat cellulaire de celle-ci. De préférence, ce produit ou cette composition est une composition alimentaire ou un complément alimentaire ou nutritionnel. Il peut être sous forme liquide ou solide. En particulier, il contient un lyophilisat de cellules ou extrait ou lysat cellulaire de celle-ci. Ce produit ou cette composition peut être sous forme de poudre, granule, gélule, capsule, ou cachet, de préférence sous forme de capsules. Alternativement, le produit ou la composition est sous forme liquide et comprend l'huile brute ou raffinée obtenue par l'un quelconque des procédés de la présente invention.

L'invention sera mieux comprise à l'aide des exemples qui suivent, lesquels se veulent illustratifs et non limitatifs.

**Exemple 1 : Etude de la production de squalène par la souche de *Schizochytrium sp.* CNCM I-4469.**

Afin de démontrer la capacité remarquable de production de squalène de la souche de *Schizochytrium sp.* CNCM I-4469, cette souche a été comparée à la souche de microalgue de référence pour la production de squalène dans la littérature, à savoir la souche *Schizochytrium mangrovei* (RCC 893, Roscoff Culture Collection, France).

Milieux de préculture et culture

La fermentation des microalgues a été conduite ici en une phase de préculture préalable avant la phase de culture / production proprement dite.

Le milieu de préculture présentait la composition décrite dans le tableau I suivant :

Tableau I

| Milieu de préculture | |
|---|---|
| Glucose | 25 g |
| Extraits de levure | 2 g |
| sel sodique de l'acide glutamique | 19 g |
| NaCl | 6 g |
| MgSO4 | 6,5 g |
| Na2SO4 | 0,1 g |
| CaCl2 | 0,1 g |
| NaHCO3 | 0,1 g |
| KH2PO4 | 2g |
| Solution concentrée en vitamines | 0,6 ml |
| Solution concentrée en oligoéléments | 0,6 ml |
| Eau déminéralisée qsq | 0,3 L |

La composition du milieu de culture / production est donné par le tableau II suivant.

Tableau II

| Milieu de production | |
|---|---|
| Glucose | 75 g |
| KH2PO4 | 9,6 g |
| Extraits de levure | 12 g |
| NaCl | 2,4 g |
| sel sodique de l'acide glutamique | 30 g |
| MqSO4 | 12 g |
| CaCl2 | 1,2 g |
| NaHCO3 | 1,2 g |
| (NH4)2SO4 | 12 g |
| KCl | 0,8 g |
| Solution concentrée en oligoéléments | 5,6 ml |
| Antimousse Clearol « FBA3107 » à 1 ml/l | 1 ml |
| Eau déminéralisée qsq | 1 L |

Composition de la solution concentrée en vitamines en g/L

| | |
|---|---|
| Vitamine B1 | 45 |
| Vitamine B6 | 45 |
| Vitamine B12 | 0,25 |

Composition la solution concentrée en oligoéléments en g/L

| | |
|---|---|
| $MnCl_2$, $2H_2O$ | 8,6 |
| $CoCl_2$, $6H_2O$ | 0,2 |
| $NiSO_4$, $6H_2O$ | 4,5 |
| $Na_2MoO_4$, $2H_2O$ | 0,15 |
| $ZnSO_4$, $7H_2O$ | 5,7 |
| $CuSO_4$, $5H_2O$ | 6,5 |
| $FeSO_4$, $7H_2O$ | 32 |

Conduite de la fermentation

La préculture a été réalisée en Erlenmeyers de 2 L munis de baffles. Le milieu de culture a été filtré après dissolution complète de ses constituants. L'inoculation a été réalisée par prélèvement de colonies de microalgues cultivées en boite de Pétri (à raison d'une oese de 10 µl). L'incubation a duré 48 heures, à une température de 25°C, sous agitation à 110 rpm (sur agitateur orbital).

La biomasse décantant (ou adhérant à la paroi), on prend bien soin de prélever 300 ml après avoir bien agité l'Erlenmeyer, ces 300 ml ont été utilisés pour ensemencer la culture.

La culture proprement dite a été réalisée de la manière suivante en Erlenmeyers de 2 L munis de baffles, à une température de 25°C, sous agitation à 110 rpm (sur agitateur orbital). Le pH initial était > 5,5.

A consommation de glucose équivalente, la durée de culture a été de 66 h pour la souche de *Schizochytrium sp.* CNCM I-4469 , et de 138 h pour la souche de *Schizochytrium mangrovei* RCC 893, traduisant une meilleure croissance de la souche *Schizochytrium sp.* CNCM I-4469.

Le tableau III suivant présente les résultats obtenus la souche *Schizochytrium* sp. CNCM I-4469 et la souche de *Schizochytrium mangrovei* RCC 893.

Tableau III

| Souche | Temps (h) | Glc consommé g/l | MS g/l | % MS culot | % squalène Brut | % squalène /MS | Squalène/L |
|---|---|---|---|---|---|---|---|
| I-4469 | 66 | 43,4 | 34,2 | 22,5 | 0,4 | 1,8 | 0,61 |
| RCC 893 | 138 | 38,2 | 20,4 | 13,5 | 0,1 | 0,4 | 0,09 |
| Glc = glucose ; MS = biomasse sèche ; Squa = squalène ; | | | | | | | |

Ainsi, il a été constaté que la souche *Schizochytrium sp.* CNCM I-4469 permet d'obtenir des teneurs en squalène de 1,8 g pour 100 g de biomasse sèche, une teneur jamais encore observée avec ces microalgues. En comparaison de la souche de référence, cette teneur est au moins 4 fois plus élevée et ce avec une durée de culture 2 fois plus courte.

Par ailleurs, une analyse de la biomasse obtenue après fermentation pour la *Schizochytrium sp.* CNCM I-4469 a permis d'observer qu'elle contenait 41,8 % en poids d'acide gras totaux, dont 17,6 % en poids d'acide docosahexaénoique (DHA).

| | | |
|---|---|---|
| **Acide Gras Totaux** | % | **41,8** |
| Ac, Laurique C12:0 | % | 0,1 |

(suite)

| | | |
|---|---|---|
| Ac, Myristique C14:0 | % | 4,8 |
| Ac, Pentadécyclique C15:0 | % | 0,4 |
| Ac, Palmitique C16:0 | % | 10,7 |
| Ac, Palmitoléique C16:1 | % | 0,2 |
| Ac, Stéarique C18:0 | % | 0,4 |
| Ac, Oléique C18:1 | % | < 0,1 |
| Ac, Linoléique **LA** C18:2 | % | 0,3 |
| Ac, Gamma Linolénique **GLA** C18:3 | % | < 0,1 |
| Ac, Alpha Linolénique **ALA** C18:3 | % | < 0,1 |
| Ac, Arachidique C20:0 | % | < 0,1 |
| Ac, Stéaridonique C18:4 | % | 0,1 |
| Ac, Gondoique C20:1 | % | < 0,03 |
| Ac, Dihomo-gamma-linolénique C20:3 | % | 0,1 |
| Ac, Arachidonique **AA** C20:4 | % | 0,1 |
| (ETE) C20:3 | % | < 0,03 |
| Ac, Béhénique C22:0 | % | < 0,1 |
| Ac, Timnodonique **EPA** C20:5 | % | 0,3 |
| Ac, Lignocérique C24:0 | % | < 0,1 |
| Ac, Osbond C22:5 | % | 6,4 |
| Ac, Nervonique C24:1 | % | < 0,1 |
| Ac, Clupanodonique **DPA** C22:5 | % | 0,2 |
| Ac, Cervonique **DHA** C22:6 | % | 17,6 |

Méthode de quantification du squalène dans la biomasse de *Schizochytrium sp.*

L'analyse a été réalisée par RMN du proton à 25°C après cassage billes de la biomasse et extraction au chloroforme/méthanol à froid. La quantification a été faite au moyen d'un étalon interne tel que décrit ci-dessous.

Les spectres ont été obtenus sur un spectromètre AVANCE III 400 (Bruker Spectrospin), opérant à 400 MHz.

Cassage de la biomasse : Peser précisément environ 200 mg de biomasse fraîche. Ajouter environ 1-1,5 cm de billes verre et 0,1 mL de Méthanol. Fermer hermétiquement le tube et agiter au moyen de l'agitateur vortex pendant au moins 5 min.

Extraction à froid : Ajouter environ 2 mg de triphényl phosphate (TPP), 0,9 mL de Méthanol et 2 mL de chloroforme. Fermer hermétiquement le tube et agiter au moyen de l'agitateur vortex pendant 1 min. Placer au réfrigérateur. Après décantation (au minimum 1 heure), récupérer délicatement la phase supérieure limpide et la transférer dans un godet verre pour évaporation à sec, à température ambiante, sous courant d'azote. Solubiliser l'extrait sec dans 0,5 mL de $CDCl_3$ et 0,1 mL de $CD_3OD$ et transférer dans un tube RMN.

Enregistrement du spectre : Effectuer l'acquisition, sans suppression de solvant, sans rotation, avec un temps de relaxation d'au moins 15 s, après les réglages appropriés de l'instrument. La fenêtre spectrale doit être au moins comprise entre -1 et 9 ppm en calibrant le spectre sur le pic de chloroforme à 7,25 ppm. Le spectre est exploité après transformation de Fourier, correction de phase et soustraction de la ligne de base en mode manuel (sans multiplication exponentielle, LB=GB=0).

Exploitation du signal : Attribuer la valeur 100 au massif du TPP ne contenant pas le signal du chloroforme entre 7,05 et 7,15 ppm (comptant pour 9 protons TPP). Intégrer la surface du signal Squalène à 1,55 ppm (singulet comptant pour 6 protons).

Calcul et expression des résultats : Les résultats ont été exprimés en pourcentage massique brut.

$$\text{Teneur} = \frac{A_s \times P_{TPP}}{6 \times 100} \times \frac{W_{TPP}}{M_{TPP}} \times M_S \times \frac{100}{PE}$$

avec

$A_s$ : surface du signal Squalène à 1,55 ppm.
$P_{TPP}$ : nombre de protons du massif TPP intégré : 9
$W_{TPP}$ : masse, en grammes, de TPP pesé
$M_{TPP}$ : masse molaire, en grammes par mole, du TPP ($M_{TPP}$=326 g/mol)
$M_s$ : masse molaire, en grammes par mole, du Squalène ($M_s$ =410 g/mol)
PE : masse, en grammes, de biomasse fraîche

**Exemple 2 : Etude du profil lipidique de la souche de *Schizochytrium sp.* CNCM I-4469.**

Le profil lipidique de cette souche (voir Tableau IV) a permis de déterminer qu'elle permet également la production d'autres composés lipidiques d'intérêt, dont l'acide docosahexaénoïque (ou DHA). Celui-ci a été réalisé sur l'huile brute extraite de la biomasse obtenue après fermentation.

Tableau IV : ***Profil lipidque de la souche en mettant en évidence les composés présentant un intérêt.***

| | | |
|---|---|---|
| Acide laurique ((dodécanoïque) 12:0) | %/com | 0,3 |
| | % surface | 0,3 |
| Acide tétradécanoïque (myristique (14:0)) | %/com | 8,4 |
| | % surface | 10,1 |
| Acide pentadécylique C15:0 | %/com | 0,6 |
| | % surface | 0,7 |
| Acide hexadécanïque (palmitique (16:0)) | %/com | 17,4 |
| | % surface | 20,9 |
| Acide hexadécènoïque (palmitoléique (16:1)) | %/com | <0,3 |
| | % surface | <0,3 |
| Acide octodécanoïque (stéarique (18:0)) | %/com | 0,4 |
| | % surface | 0,5 |
| Acide octadécénoïque (oléique (18:1 w-9)) | %/com | <0,1 |
| | % surface | |
| Acide octadécadènoïque (linoléique (LA) (18:2 w-6)) | %/com | <0,3 |
| | % surface | <0,3 |
| Acide octadécatrïènoïque (alpha linolenique (ALA) (18:3 w-3)) | %/com | <0,1 |
| | % surface | |
| Acide octadécatrïènoïque (gamma linolenique (GLA) (18:3 w-6)) | %/com | <0,3 |
| | % surface | <0,3 |
| Acide octadecatetraènoïque ({stéaridonique) 18:4 w-3) | %/com | <0,3 |
| | % surface | 0,3 |
| Acide eïcosanoïque (arachidique (20:0) | %/com | <0,1 |
| | % surface | |

(suite)

| | | |
|---|---|---|
| Acide eïcosènoïque ((gondoique) 20:1 w-9) | %/com | **<0,1** |
| | % surface | |
| Acide eicosatriénoïque ((ÉTÉ) 20:3 w-3) | %/com | **<0,1** |
| | % surface | |
| Acide eïcosatriènoïque (dihomo gamma linolénique (20:3 w-6)) | %/com | **0,3** |
| | % surface | **0,4** |
| **Acide arachidonique (ARA)(20:4w-6)** | **%/com** | **0,4** |
| | **% surface** | **0,5** |
| Acide eicosapentaénoïque (EPA)(20:5w-3) | %/com | **1,0** |
| | % surface | **1,2** |
| Acide docosanoïque (béhénique (22:0)) | %/com | **<0,3** |
| | % surface | **0,3** |
| Acide docosa pentaénoïque ((clupanodonique) 22:5 w-3) | %/com | **0,4** |
| | % surface | **0,5** |
| Acide docosapentaènoïque (osbond (22:5 w-6)) | %/com | **12,2** |
| | % surface | **14,6** |
| **Acide Docosahexaenoique (DHA)(22:6w-3)** | **%/com** | **37,6** |
| | **% surface** | **45,1** |
| Acide tétracosanoïque (lignocérique (24:0)) | %/com | **<0,3** |
| | % surface | **0,3** |
| Acide nervonique ((tétracosènoïque) 24:1 w-9) | %/com | **<0,3** |
| | % surface | **<0,3** |
| Squalène | % | **5,8** |
| Acides gras non identifiés | %/com | |
| | % surface | **<3,9** |

Méthode de préparation du profil lipidique

Les acides gras ont été déterminés par chromatographie en phase gazeuse sous la forme d'esters méthyliques après transestérification au méthanol chlorhydrique et extraction au chloroforme. Les résultats sont exprimés en distribution, en % ; l'analyse est réalisée par la méthode de normalisation interne.

Un chromatographe (Type VARIAN 3800) équipé d'un injecteur split-splitless muni d'un tapfocus liner et d'un détecteur à ionisation de flamme a été utilisé.

Une solution d'étalon interne à environ précisément 0.5 mg de méthylheptadécanoate par mL de méthanol a été préparée. Le méthylheptadécanoate a servi de repère chromatographique.

Dans un tube de 6 mL, on a pesé environ précisément 30 mg d'échantillon séché au préalable. On a ajouté à la pipette à 2 traits 1 mL de la solution d'étalon interne puis 2 mL de méthanol chlorhydrique 3N. On a ensuite bouché et placé au bain à sec thermostaté à 110°C pendant 4 h.

Après refroidissement, on a ajouté environ 0.5 mL d'eau et 0.5 mL d'eau saturée en chlorure de sodium, extraire par 3 fois 1 mL de chloroforme. On a récupéré les phases chloroformiques dans un tube de 6 mL en les séchant sur une colonne contenant du sulfate de sodium. On a concentré sous courant d'azote jusqu'à 1 mL environ et injecté.

La distribution de chaque acide gras (i), en %, a été obtenu par le ratio de la surface du pic de cet acide gras par rapport à la somme des surfaces de tous les pics repérés sur le chromatogramme, de l'acide laurique (C12:0) au DHA (C22:6 $\Delta$4c, 7c, 10c, 13c, 16c, 19c) inclus, en excluant le pic du méthylheptadécanoate.

**Revendications**

1. Souche de *Schizochytrium sp.* déposée le 14 avril 2011 auprès de la CNCM sous le numéro I-4469.

2. Souche de *Schizochytrium sp.* **caractérisée en ce qu'**elle est capable de produire des teneurs en squalène supérieures ou égales à 1 g pour 100 g de biomasse sèche et conserve la capacité de production de squalène de la souche I-4469, et qu'elle est obtenue à partir de la souche selon la revendication 1 par mutagenèse ou par transformation génique.

3. Méthode de production des composés lipidiques d'intérêt comprenant une culture de la souche selon la revendication 1 ou 2 et la récupération de la biomasse riche en composés lipidiques d'intérêt et, facultativement, la récolte des composés lipidiques d'intérêt.

**4.** Méthode selon la revendication 3, dans laquelle les composés lipidiques d'intérêt sont le squalène ou l'acide docosahexaénoïque (ou DHA), de préférence le squalène.

**5.** Utilisation de la souche selon la revendication 1 ou 2 pour produire des composés lipidiques d'intérêt, tel le squalène ou l'acide docosahexaénoïque (ou DHA).

**6.** Produit ou composition comprenant la souche selon la revendication 1 ou 2.

**7.** Produit ou composition selon la revendication 6, **caractérisé en ce qu'**il est riche en squalène.

**8.** Produit ou composition selon la revendication 6 ou 7, **caractérisé en ce qu'**il s'agit d'un produit alimentaire ou complémentaire alimentaire.

**9.** Méthode de préparation d'une souche de *Schizochytrium sp.* capable de produire des teneurs en squalène supérieures ou égales à 1 g pour 100 g de biomasse sèche et conservant la capacité de production de squalène de la souche I-4469, comprenant la mutagenèse ou la transformation génique de la souche selon la revendication 1.

**Patentansprüche**

**1.** *Schizochytrium sp.* Stamm, hinterlegt am 14. April 2011 bei der CNCM unter der Nummer I-4469.

**2.** *Schizochytrium sp.* Stamm, **dadurch gekennzeichnet, dass** er in der Lage ist, Squalen mit einem Gehalt von mehr als oder gleich 1 g pro 100 g Biotrockenmasse zu produzieren, und die Fähigkeit des Stamms 1-4469 zur Produktion von Squalen konserviert, und dass er ausgehend von dem Stamm gemäß Anspruch 1 mittels Mutagenese oder mittels Gentransformation erhalten wird.

**3.** Verfahren zur Produktion der Lipidverbindungen von Interesse, umfassend Kultivierung des Stamms gemäß Anspruch 1 oder 2 und Wiedergewinnung der Biomasse, die reich an Lipidverbindungen von Interesse ist, und gegebenenfalls Ernte der Lipidverbindungen von Interesse.

**4.** Verfahren gemäß Anspruch 3, wobei die Lipidverbindungen von Interesse Squalen oder Docosahexaensäure (oder DHA), vorzugsweise Squalen, sind.

**5.** Verwendung des Stamms gemäß Anspruch 1 oder 2 zur Produktion der Lipidverbindungen von Interesse, wie Squalen oder Docosahexaensäure (oder DHA).

**6.** Produkt oder Zusammensetzung, umfassend den Stamm gemäß Anspruch 1 oder 2.

**7.** Produkt oder Zusammensetzung gemäß Anspruch 6, **dadurch gekennzeichnet, dass** es/sie reich an Squalen ist.

**8.** Produkt oder Zusammensetzung gemäß Anspruch 6 oder 7, **dadurch gekennzeichnet, dass** es sich um ein Nahrungsmittel oder Nahrungsergänzungsmittel handelt.

**9.** Verfahren zur Herstellung eines *Schizochytrium sp.* Stamms, der in der Lage ist, Squalen mit einem Gehalt von mehr als oder gleich 1 g pro 100 g Biotrockenmasse zu produzieren, und der die Fähigkeit des Stamms 1-4469 zur Produktion von Squalen konserviert, umfassend die Mutagenese oder die Gentransformation des Stamms gemäß Anspruch 1.

**Claims**

**1.** A *Schizochytrium sp.* strain deposited on April 14, 2011, with the CNCM under number I-4469.

**2.** A *Schizochytrium sp.* strain **characterized in that** it is capable of producing squalene contents greater than or equal to 1 g per 100 g of dry biomass, that it keeps the squalene production capacity of the strain CNCM I-4469 and that it is obtained from the strain as claimed in claim 1 by mutagenesis or by gene transformation.

3. A method for producing lipid compounds of interest, comprising culturing the strain as claimed in claim 1 or 2 and recovering the biomass rich in lipid compounds of interest and, optionally, harvesting the lipid compounds of interest.

4. The method as claimed in claim 3, wherein the lipid compounds of interest are squalene or docosahexaenoic acid (or DHA), preferably squalene.

5. Use of the strain as claimed in claim 1 or 2 for producing lipid compounds of interest, such as squalene or docosahexaenoic acid (or DHA).

6. A product or composition comprising the strain as claimed in claim 1 or 2.

7. The product or composition as claimed in claim 6, **characterized in that** it is rich in squalene.

8. The product or composition as claimed in claim 6 or 7, **characterized in that** it is a food product or food supplement product.

9. A method for preparing a *Schizochytrium sp.* strain capable of producing squalene contents greater than or equal to 1 g per 100 g of dry biomass and keeping the squalene production capacity of the strain CNCM I-4469, comprising mutagenesis or gene transformation of the strain as claimed in claim 1.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010023551 A

**Littérature non-brevet citée dans la description**

- **BHATTACHARJEE, P. et al.** *World J. Microb. Biotechnol.,* 2001, vol. 17, 811-816
- **LEWIS et al.** *Mar. Biotechnol.,* 2001, 439-447
- **JIANG et al.** *J. Agric. Food Chem.,* 2004, vol. 52, 1196-1200
- **LI et al.** *J. Agric. Food Chem.,* 2009, vol. 57, 4267-4272
- **QIAN LI et al.** *J. Agric. Food Chem.,* 2009, vol. 57, 4267-4272
- **LEWIS et al.** *Mar. Biotechnol.,* 2001, vol. 3, 439-447
- **G. CHEN et al.** *New Biotechnology,* 2010, vol. 27-4, 382-389
- **K. W. FAN et al.** *World J. Microbiol. Biotechnol.,* 2010, vol. 26-3, 1303-1309
- Optimisation of culture conditions of thraustochytrid, Aurantiochytrium sp. Strain 18W-13a for squalene production. **NAKAZAWA et al.** The 1 st Asia Oceania algae innovation Summit. University of Tsukuba, 13 Décembre 2010
- **TSUI et al.** *Molecular Phylogenetics and Evolution,* 2009, vol. 50, 129-140
- **QIAN et al.** *Journal of Agricultural and Food Chemistry,* 2009, vol. 57, 4267-4272

SEQUENCE LISTING

<110> ROQUETTE FRERES

<120> NOUVELLE SOUCHE DE MICROALGUE PRODUCTRICE DE SQUALENE

<130> B1187PC

<160> 1

<170> Patent In version 3.3

<210> 1
<211> 479
<212> DNA
<213> Schizochytrium sp

<400> 1

```
gagggtttta cattgctctc attccaatag caagacgcga agcgccccgc attgatattt
60

ctcgtcacta cctcgtggag tccacattgg gtaatttacg cgcctgctgc cttccttgga
120

tgtggtagcc gtctctcagg ctccctctcc ggagtcgagc cctaactccc cgtcacccgt
180

tatagtcacc gtaggccaat accctaccgt cgacaactga tggggcagaa actcaaacga
240

ttcatcgctc cgaaaagcga tctgctcaat tatcatgact caccaagaga gttggcttag
300

acctaataag tgcggccctc cccgaaagtc gggcccgtac agcacgtatt aattccagaa
360

ttactgcagg tatccgtata aaggaactac cgaagggatt ataactgata taatgagccg
420

ttcgcagttt cacagtataa ttcgcttata cttacacatg catggcttag tctttgaga
479
```